# EUROPEAN PATENT APPLICATION

(11) **EP 4 470 999 A1**
(43) Date of publication of application: **04.12.2024**
(21) Application number: 23176697.3
(22) Date of filing: 01.06.2023
(51) Int. Cl.: C07C 4/22, C07C 15/46, C08J 11/02, C08J 11/12

(54) **A PROCESS AND PLANT FOR RECYCLING POLYSTYRENE WASTE CONTAINING AT LEAST ONE ORGANIC HALOGEN CONTAINING FLAME RETARDANT**

(71) Applicant: Sulzer Management AG, 8401 Winterthur (CH)
(72) Inventor: YARULINA, Irina, 8400 Winterthur (CH); HOFFMANN, Luis, 8406 Winterthur (CH); TROMMSDORFF, Ulla, 8057 Zürich (CH); D'ELIA, Marco, 8910 Affoltern am Albis (CH); WALKER, Claudio, 8405 Winterthur (CH)
(74) Representative: Henkel & Partner mbB

(57) **Abstract**

The present invention relates to a process for recycling polystyrene waste containing at least one organic halogen containing compound by converting polystyrene to styrene comprising the steps of:
a) providing a melt of polystyrene waste containing at least one organic halogen containing compound,
b) subjecting the melt provided in step a) to at least one devolatilization step so as to obtain a devolatilized composition,
c) subjecting the devolatilized composition obtained in step b) to at least one pyrolysis step so as to obtain a pyrolyzed composition,
d) subjecting the pyrolyzed composition to at least one distillation step so as to obtain a purified styrene composition.

## Description

The present invention relates to a process for recycling polystyrene waste containing at least one organic halogen containing compound, such as in particular an organic halogen containing flame retardant, such as 1,2,5,6,9,10-hexabromo-cyclododecane, by converting polystyrene to styrene and to a plant for performing this process.

Polystyrene is one of the most widely used plastics worldwide, among others because it is excellently moldable to the desired form, such as by injection molding or extrusion. Polystyrene is for instance used as jewel cases, as food packagings, for instance yoghurt cups, as plastic housings, in electronic components, for instance in switches and as dielectric layers, as petri dishes, as test tubes or the like. In addition, on account of its excellent thermal insulation properties polystyrene foam is widely used for instance as building insulation materials, such as in insulating concrete forms and structural insulated panel building systems. One important drawback of polystyrene is its easy flammability, whereupon polystyrene releases large amounts of black smoke upon burning. Therefore, polystyrene and in particular polystyrene foam, such as expanded polystyrene (EPS) or extrude polystyrene (XPS), typically contains flame retardants, in order to drastically reduce the flammability of the polystyrene or polystyrene foam, respectively, which is a prerequisite for its use as building material. In particular in the past, but also today, as flame retardant organic halogen containing compounds, such as organic bromine containing compounds, for instance 1,2,5,6,9,10-hexabromo-cyclododecane (HBCD), were/are used.

The recycling of polystyrene waste becomes more and more important. First of all, polystyrene is a homopolymer of styrene or a copolymer containing styrene comonomer, which is typically produced by (co)polymerizing styrene. However, styrene is typically obtained from raw oil or from hydrocarbon streams deriving from raw oil, respectively, wherefore is not only from an economical viewpoint, but in particular also from an ecological viewpoint advantageous to recover the raw material styrene from polystyrene waste. Furthermore, the disposal of polystyrene and in particular of polystyrene foam is challenging and expensive on account of the presence of contaminants of different nature within the polystyrene waste. The same is valid for the recycling of polystyrene to styrene, since the additives and in particular flame retardants, such as brominated flame retardants, for instance hexabromocyclododecane, contained therein are difficult to be completely removed from the polystyrene so that the resulting styrene composition usually contains these flame retardants or decomposition products thereof being generated during the recycling process. For instance, a plurality of known processes for recycling of polystyrene bases on the pyrolysis of polystyrene to styrene, during which organic halogen containing compounds contained in the polystyrene as flame retardant, such as hexabromocyclododecane, are decomposed to the respective halogen, such as bromine, hydrogen halogenide and/or halogen containing hydrocarbon side-products. However, the presence of these flame retardants or decomposition products, especially halogens, such as bromine, or hydrogen halogenides, such as hydrogen bromide, in the recycled styrene composition is a problem, because the flame retardants or decomposition products, respectively, cause - even if the flame retardants or decomposition products, respectively, are contained in the recycled styrene composition in small amounts - during the use of the recycled styrene composition, such as in a (co)polymerization reaction a corrosion and/or fouling of the piping and reactor equipment and/or a poisoning of the used catalyst. Even if such organic halogen containing flame retardants, such as in particular HBCD, have been restricted in their use in the past, a huge amount of polystyrene products having been produced before the legal regulations came into force are still in use and need to be recycled in the future. Moreover, certain country-specific exemptions exist, which allow the application of HBCD in EPS and XPS utilized in buildings insulations. All in all, an efficient recycling of polystyrene waste including organic halogen containing compounds as flame retardants is important and such an efficient recycling of polystyrene waste requires a complete removal of organic halogen containing compounds contained therein as flame retardants as well as of possible decomposition products, such as halogen, hydrogen halogenide and/or halogen containing hydrocarbons being formed during the recycling process from the flame retardants.

In view of this, the object underlying the present invention is to provide a process for recycling polystyrene waste containing at least one organic halogen containing compound by converting polystyrene to styrene, which leads to pure styrene, which contains, if at all, only minimal, non-disturbing amounts of organic halogen containing compounds and decomposition products resulting therefrom, such as bromine and/or hydrogen bromide, wherein the process is nevertheless characterized by low operational and capital expenditures.

In accordance with the present invention, this object is solved by providing a process for recycling polystyrene waste containing at least one organic halogen containing compound by converting polystyrene to styrene comprising the steps of:
a) providing a melt of polystyrene waste containing at least one organic halogen containing compound,
b) subjecting the melt provided in step a) to at least one devolatilization step so as to obtain a devolatilized composition,
c) subjecting the devolatilized composition obtained in step b) to at least one pyrolysis step so as to obtain a pyrolyzed composition,
d) subjecting the pyrolyzed composition to at least one distillation step so as to obtain a purified styrene composition.

This solution bases on the finding that by subjecting a melt of the polystyrene waste to be recycled and containing at least one organic halogen containing compound to at least one devolatilization step, before the devolatilized composition obtained thereby is pyrolyzed, wherein the pyrolyzed composition obtained thereby is subjected to at least one distillation step and optionally thereafter to a further purification step, such as one or more crystallization steps, a pure styrene composition with a styrene content of more than 98.8% by weight of styrene, of at least 99.8% by weight or even of at least 99.94% by weight of styrene is obtained, which contains no or, if at all, only very low amounts of at least one organic halogen containing compound and possible decomposition products, such as halogen, hydrogen halogenide and/or halogen containing hydrocarbons. Without wishing to be bound to any theory, it is considered that organic halogen containing compounds contained in the polystyrene as flame retardant, such as hexabromocyclododecane (HBCD), as well as decomposition products generated during the devolatilization, such as halogen (for instance bromine), hydrogen halogenide (for instance hydrogen bromide) and/or halogen containing hydrocarbon side-products, are efficiently and completely or at least essentially completely removed from the polymer melt, before the devolatilized polymer melt is subjected to the pyrolysis reaction. In particular, the use of solvents, additives and/or catalysts used in prior art process for removing these contaminants is not required in the process in accordance with the present invention, so that the disadvantages thereof, such as additional separation equipment and catalyst/additive recovery systems leading to additional operational and apparatus costs, are not necessary. In addition, since organic halogen containing compounds are removed in the process, maintenance costs due to corrosion in the recycling plant are prevented and the process may be performed with a reactor containing comparable cheap internals. All in all, the process in accordance with the present invention does not only lead to very pure styrene, but is also characterized by low operational and capital expenditures.

Pyrolysis means in accordance with the present invention any kind of thermal decomposition.

In accordance with the present invention, in step a) a melt of polystyrene waste containing at least one organic halogen containing compound is provided. For instance, the polystyrene waste used in step a) contains 0.5 to 15% by weight and preferably 2 to 4% by weight of at least one organic halogen containing flame retardant. The present invention is in particular suitable to use polystyrene waste containing as organic halogen containing compound at least one bromine containing flame retardant and more preferably tetrabromobisphenol A, a brominated polyacrylate, a polybrominated diphenyl ether or a hexabromocyclododecane, such as 1,2,5,6,9,10-hexabromocyclododecane. The polystyrene waste may contain non-foamed polystyrene or foamed polystyrene, such as EPS or XPS.

The present invention is not particularly limited concerning the kind, how in step a) the polystyrene waste is molten to a melt. Good results are in particular obtained, when the polystyrene waste containing at least one organic halogen containing compound is molten in step a) in an extruder, such as in a single screw extruder or in a twin-screw extruder. An extruder does not only pre-heat and melt the polystyrene waste, but also mixes it in a continuous manner. Good results are in particular obtained, when the polystyrene waste is heated in the extruder to a temperature of 150 to 250°C and preferably to 180 to 220°C. The polystyrene waste may be fed into the extruder in any form, such as in the form of chips, flakes, pellets, powder or the like.

In accordance with a particular preferred embodiment of the present invention, the melt of polystyrene waste containing at least one organic halogen containing compound being provided in step a) is subjected in step b) to at least one devolatilization step b), which is preferably performed at a temperature of 180 to 260°C and more preferably at a temperature of 200 to 260°C. Thereby, the at least one organic halogen containing compound is reliably evaporated and/or decomposed to one or more gaseous compounds, such as halogen and/or hydrogen halogenide, carbon dioxide, carbon monoxide and brominated polyaromatics, and removed as gaseous composition from the remaining, devolatilized polystyrene melt.

In a further development of the idea of the present invention, it is suggested that the residence time of the melt in the at least one devolatilization step is 5 to 120 minutes, preferably 10 to 80 minutes, if the temperature is 210 to 260°C, and preferably 10 to 30 minutes, if the temperature is 230 to 250°C. Such a residence time contributes to a sufficient decomposition and removal of the at least one organic halogen containing compound and the decomposition products formed therefrom from the polystyrene melt.

The present invention is not particularly limited concerning the pressure during the at least one devolatilization step. Good results are in particular obtained, when the at least one devolatilization step is performed at ambient pressure or slightly below ambient pressure, such as preferably at 0.1 to 20 kPa absolute pressure.

The at least one devolatilization step may be performed in a devolatilization vessel comprising at least one distributor and/or at least one internal selected from trays, structured packings and random packings. However, the at least one devolatilization step also satisfyingly works, if no distributor and/or no internal is comprised in the devolatilization vessel.

In accordance with a particular preferred embodiment of the present invention, the melt of polystyrene waste containing at least one organic halogen containing compound provided in step a) is mixed in at least one static mixer, before or during the at least one devolatilization step. Thereby the polystyrene melt is homogenized, before it is devolatilized. Preferably, the polystyrene melt is mixed before the at least one devolatilization step, or, if the polystyrene melt is mixed during the at least one devolatilization step, it is mixed at shortly after the beginning of the at least one devolatilization step. Good results are in particular obtained, when the melt of polystyrene waste containing at least one organic halogen containing compound provided in step a) is mixed in at least one static mixer with heat exchanging capabilities before or during the at least one devolatilization step. Thereby, the temperature of the polystyrene melt may be increased in a rapid, homogeneous, and highly controlled manner, before or at the beginning of the start of the devolatilization.

Static mixer means any mixer, which does not comprise any moving part and does in particular not comprise any rotating part and any such mixer may be used in the present invention. Static mixers usually produce a mixing effect by generating a turbulent flow due to static, i.e. non-moving elements, such as plates, bars, crossbars, baffles, helically formed deflection means, grids and the like. If these elements are hollow, heat transfer medium may be flowed through the elements so that the respective static mixer has heat exchanging capabilities. Suitable examples for static mixers, are x-type static mixers, spiral/helical-type static mixers, quattro-type static mixers, baffle plate-type static mixers, turbulator strips-type static mixers and any combination of two or more of the abovementioned mixer types. X-type static mixers comprise deflection means in the form of bars, crossbars, plates or the like having in a plan view and/or side view and/or cross-sectional view a x-like form. Such x-type static mixers are described for instance in WO 2010/066457 A1, EP 1 206 962 A1, EP 2 158 027 B1 and EP 0 655 275 B1 and are commercially available from Sulzer Chemtech Ltd, Winterthur, Switzerland under the tradenames SMX, SMXL and SMX plus as well as from Fluitec, Neften-bach, Switzerland under the tradename CSE-X. Spiral/helical-type static mixers have a helically formed deflection means and are described for instance in US 3,743,250 A, whereas quattro-type static mixers comprise deflection means forming chamber-like mixing sections and are described for instance in EP 2 548 634 B1 and in EP 0 815 929 B1. While baffle plate-type static mixers comprise usually longitudinal deflection means and are described for instance in EP 1 510 247 B1 and in US 4,093,188 A, turbulator strip-type static mixers comprise in a tube a plurality of elongated strips, each of which being formed by a series of alternating deflection panels successively joined together by for example substantially triangular bridging portions with the strips being held together and anchored substantially on the axis of the tube by alternate ones of the bridging portions and the other bridging sections being disposed adjacent the inner wall of the tube and are described for instance in US 4,296,779 A. Other suitable static mixers are distributed from Sulzer Chemtech AG under the tradenames CompaX, SMI, KVM, SMV and GVM and from Stamixco AG, Wollerau, Switzerland under the tradename GVM. In view of the above, it is preferred that at least one and more preferred that all of the at least one static mixer of the at least two distributors is selected from the group consisting of x-type static mixers, spiral/helical-type static mixers, quattro-type static mixers, baffle plate-type static mixers, turbulator strips-type static mixers and any combination of two or more of the above mentioned mixer types.

The gaseous composition generated during the at least one devolatilization step, is removed from the vessel, in which the at least one devolatilization step is performed. It may be processed, such as by subjecting it to a caustic wash for instance with an acidic gas, such as with hydrogen bromide, and/or by condensing it to a liquid light hydrocarbon composition.

In order to further reduce the amount of organic halogen containing compounds in the polystyrene melt, the devolatilized composition may be subjected to at least one further devolatilization step, if necessary. The at least one further devolatilization step may be for example performed under the same or similar temperature and pressure conditions and with the same or similar residence time as in the above described first devolatilization step.

In accordance with the present invention, the devolatilized composition is then subjected to at least one pyrolysis step or thermal decomposition, respectively, so as to obtain a pyrolyzed composition or thermally decomposed composition, respectively. Good results are in particular obtained, when the at least one pyrolysis step is performed at a temperature of 350 to 1 ,000°C and more preferably at a temperature of 450 to 650°C. Thereby, the polystyrene contained in the devolatilized composition is decomposed to styrene.

The residence time of the pyrolyzed composition in the pyrolysis reactor is preferably 0.1 to 15 seconds.

In a further development of the idea of the present invention, it is proposed that the at least one pyrolysis step is performed in an isothermal reactor or in an adiabatic reactor. For instance, the pyrolysis step may be performed in a rotary kiln, in a pyrolysis reactor (batch) or in fluidized bed reactor. Good results are in particular obtained, when a fluidized bed is used as isothermal reactor.

The pyrolysis is preferably performed in an inert gas atmosphere, such as in nitrogen or a noble gas, or in the atmosphere of the formed product.

The pyrolysis products or pyrolyzed composition, respectively, preferably contains 60 to 85% by weight of styrene, at most 15% by weight of a gaseous composition and 5 to 20% by weight of dimers or trimers of styrene.

In accordance with a particular preferred embodiment of the present invention, the pyrolyzed composition obtained in step c) is rapidly cooled, in order to prevent further side-reactions and in order to maximize the styrene monomer fraction in the pyrolyzed composition, before it is subjected in step d) to at least one distillation step. More specifically, it is preferred that the pyrolyzed composition obtained in step c) is cooled with a cooling rate of at least 400°C/minute to a temperature of at most 200°C and preferably of 40 to 200°C, before it is subjected in step d) to at least one distillation step. Good results are in particular obtained, when the cooling is performed in a spray condenser or in a shell-and-tube heat exchanger.

In accordance with the present invention, the pyrolyzed composition is subjected in step d) to at least one distillation step so as to obtain a purified styrene composition. Good results are in particular obtained, when the pyrolyzed composition is subjected in step d) to one to three and more preferably to one or two distillation steps.

In accordance with a first variant, the pyrolyzed composition is subjected to two distillation steps, wherein the pyrolyzed composition is distilled in the first distillation step into an overhead composition comprising C₇₋-hydrocarbons and into a bottom composition, wherein the bottom composition is distilled in the second distillation step to an overhead composition comprising purified styrene and to a bottom composition containing C9+-hydrocarbons, such as dimer of styrene.

Preferably, the temperature in each of the distillation columns is adjusted to 30 to 145°C and more preferably to 40 to 110°C, whereas the pressure in each of the distillation columns is preferably adjusted to ambient pressure or below ambient pressure, such as to 10 to 50 kPa absolute pressure.

The purified styrene composition obtained in this variant contains preferably more than 98.8% by weight, more preferably at least 99.2% by weight and yet more preferably at least 99.5% by weight, such as at least 99.8% by weight of styrene. In turn, the content of organic halogen containing compounds and decomposition products thereof, such as halogen, hydrogen halogenide and halogenated hydrocarbons, in the purified styrene composition is at most 5 ppm and preferably at most 2 ppm.

In accordance with an alternative, second variant, the pyrolyzed composition obtained in step c) is subjected in step d) to three distillation steps, wherein at least the first two of the three distillation steps are performed as extractive distillations. Suitable extractive agents are sulfolane, cyrene and arbitrary combinations of two or more of the aforementioned extractive agents.

Also the purified styrene composition obtained in this variant contains preferably more than 98.8% by weight, more preferably at least 99.2% by weight and yet more preferably at least 99.5% by weight, such as at least 99.8% by weight of styrene. In turn, the content of organic halogen containing compounds and decomposition products thereof, such as halogen, hydrogen halogenide and halogenated hydrocarbons, in the purified styrene composition is at most 5 ppm and preferably at most 2 ppm.

In accordance with an alternative, third variant, the pyrolyzed composition obtained in step c) is subjected in step d) to a distillation step in a dividing-wall distillation column. Concerning the kind of dividing-wall distillation column, the present invention is not particularly restricted. Thus, any distillation column comprising a dividing wall, which separates at least a longitudinally extending section of the dividing wall, seen in its cross-section, into two sub-sections may be used. Preferably, the dividing wall is arranged at least essentially vertical downwards in the dividing-wall distillation column. At least essentially vertically downwards means in accordance with the present invention that the angle between the dividing wall and the length axis of the dividing-wall distillation column or vertical direction, respectively, is at most 20°, preferably at most 10°, more preferably at most 5° and most preferably 0°.

In accordance with a particularly preferred embodiment of the present invention, the composition to be pyrolyzed is fed in step a) into a middle dividing-wall distillation column and distilled therein into an overhead composition comprising C₇₋-hydrocarbons, into a side composition of purified styrene and into a bottom composition containing C₉₊-hydrocarbons, such as dimer and/or trimer of styrene. A middle dividing-wall distillation column is according to the present invention defined as a dividing-wall distillation column, which comprises a dividing wall extending from a point being located below the top of the distillation column at least essentially vertically downwards to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the dividing wall and a second middle subsection being located on the opposite side of the dividing wall. Good results are in particular obtained, when the dividing wall extends, seen from the bottom to the top of the dividing-wall distillation column, from a point being located at 10 to 45% of the distance from the bottom to the top of the dividing-wall distillation column to a point being located at 50 to 90% of the distance from the bottom to the top of the dividing-wall distillation column and preferably from a point being located at 25 to 40% of the distance from the bottom to the top of the dividing-wall distillation column to a point being located at 60 to 80% of the distance from the bottom to the top of the dividing-wall distillation column. Moreover, it is preferred that the dividing wall extends, seen from the bottom to the top of the dividing-wall distillation column, over 5 to 90%, more preferably over 20 to 80% and most preferably over 30 to 70% of the distance from the bottom to the top of the dividing-wall distillation column.

In a further development of the idea of the present invention, it is suggested that the dividing wall subdivides the middle section of the dividing-wall distillation column, seen in cross-section of the dividing-wall distillation column, in about two equally sized subsections or halves, respectively. Therefore, it is preferred that one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column. For instance, one subsection covers 43% and the other 57% of the total area of the cross-section or both subsections cover each exactly 50% of the total area of the cross-section. If the total area of the cross-section of the middle section varies over the axial length of the middle section, the above numeric values are related to the average total area of the cross-section of the middle section.

Preferably, the distillation is performed in the middle dividing-wall distillation column at a temperature of 30 to 145°C and at a pressure of X to Y MPa and more preferably at a temperature of X to Y°C and at a pressure of X to Y MPa.

The side stream may or may not subjected to a second distillation step (regular).

Also the purified styrene composition obtained in this variant contains preferably more than 98.8% by weight, more preferably at least 99.2% by weight and yet more preferably at least 99.5% by weight, such as at least 99.8% by weight of styrene. In turn, the content of organic halogen containing compounds and decomposition products thereof, such as halogen, hydrogen halogenide and halogenated hydrocarbons, in the purified styrene composition is at most 5 ppm and preferably at most 2 ppm.

In order to even increase the purity degree of the styrene product, the purified styrene composition obtained in the at least distillation step may be subjected to at least one crystallization step and particularly preferably to at least one melt crystallization step.

The present invention is neither particularly limited concerning the kind of at least melt crystallization step nor concerning the number of melt crystallization steps.

Good results are in particular obtained, when the at least one melt crystallization step comprises at least one melt crystallization step being selected from the group consisting of static crystallization steps, suspension crystallization steps and crystallization step crystallization steps. Each of the aforementioned crystallization techniques may comprise one to twelve, preferably one to ten, more preferably one to five and most preferably one to three melt crystallization steps.

In accordance with a particular preferred embodiment of the present invention, the at least one melt crystallization step comprises at least one falling film crystallization step. During a falling film crystallization step, melt to be crystallized flows along a cooled surface downwards, such as along the inside of a cooled tube, which allows crystals to grow from the falling film of melt on the inside surface of the tube, which is cooled by a falling film of a cooling agent co-currently flowing on the outside surface of the tube. High and very reproducible transfer rates are achieved on both side of the tube, wherein the resulting shear at the crystal/liquid interface transports impurities rapidly into the bulk of the melt. Preferably, the falling film crystallizer used for the melt crystallization step(s) contains a plurality of vertical tubes, in which the crystal layers grow as cylindrical shells, a collection vessel beneath the tubes as well as a circulating pump. Before the start of a crystallization step, the collection vessel is filled with a batch of the melt to be crystallized. The circulating pump is then started to irrigate the tubes, while coolant temperature ramping is initiated. The melt circulation rate is adjusted to a high value compared to the rate of crystal deposition so that conditions of temperature and composition are approximately uniform across the length of the tubes. The temperature is ramped down at a constant rate until the collecting vessel level drops to a preset value, indicating the desired amount of product is deposited within the tubes. At this point, melt circulation is discontinued.

During each crystallization step, a styrene enriched crystallized fraction and a styrene depleted residue fraction are obtained, wherein the remaining liquid is removed from the crystallization step as styrene depleted residue fraction after termination of the crystallization in the crystallization step. Thereafter the crystal layer obtained in the crystallization step is molten and withdrawn as styrene enriched crystallized fraction from the crystallization step.

In a further development of the idea of the present invention, it is suggested that before melting the crystal layer obtained in the crystallization step, one or more sweating steps of the crystal layer are carried out so as to obtain one or more sweating fractions and a purified crystal layer, wherein preferably at least a portion of the first sweating fraction obtained thereby is fed to the remaining liquid which has been removed as styrene depleted residue fraction. Sweating is achieved by increasing the temperature of the crystals to a numeric value being just below the melting point of styrene, such as 0.1 to 2°C below the melting point of styrene, in order to liquefy impurities and assist further draining.

Good results are in particular obtained, when at least one and preferably all of the at least one melt crystallization steps is/are performed at a temperature of -200°C to 30°C, preferably at a temperature of -140°C to 0°C and more preferably at a temperature of -100°C to -30°C.

In accordance with a particular preferred embodiment of the present invention, the purified styrene composition obtained in the at least one distillation step is subjected to at least one dynamic melt crystallization step and to at least one static melt crystallization step, wherein preferably first at least one dynamic melt crystallization step is performed and thereafter at least one static melt crystallization step is performed. Good results are in particular obtained, when the purified styrene composition obtained in the at least one distillation step is subjected to one to ten and preferably one to five falling film melt crystallization steps and thereafter to one to ten and preferably one to five static melt crystallization steps.

In accordance with a particular preferred embodiment of the present invention, the purified styrene composition contains after the at least one crystallization step of more than 98.8% by weight, more preferably at least 99.2% by weight, yet more preferably at least 99.5% by weight, still more preferably at least 99.7% by weight, even more preferably at least 99.9% by weight and most preferably of at least 99.94% by weight of styrene. In turn, the content of organic halogen containing compounds and decomposition products thereof, such as halogen, hydrogen halogenide and halogenated hydrocarbons, in the purified styrene composition is at most 5 ppm and preferably at most 2 ppm.

In accordance with a further aspect, the present invention relates to a plant for recycling polystyrene waste containing at least one organic halogen containing compound by converting polystyrene to styrene comprising:
i) at least one devolatilization vessel comprising an inlet for a melt of polystyrene waste containing at least one organic halogen containing compound, an outlet for gaseous composition and an outlet for devolatilized composition,
ii) at least one pyrolysis reactor comprising an inlet being connected with the outlet for devolatilized composition of the at least one devolatilization vessel i) and an outlet for pyrolyzed composition, and
iii) at least one distillation column comprising an inlet being connected directly or indirectly with the outlet for the pyrolyzed composition of the at least one pyrolysis reactor, an overhead outlet and a bottom outlet.

Preferably, the plant further comprises an extruder, preferably a single screw extruder or twin-screw extruder, which is arranged upstream of the at least one devolatilization vessel. The extruder comprises an inlet for polystyrene waste containing at least one organic halogen containing compound and an outlet for melt of polystyrene waste containing at least one organic halogen containing compound, wherein the outlet for melt of the extruder is connected with the inlet of the at least one devolatilization vessel.

In addition, it is preferred that the plant further comprises a cooler comprising an inlet being connected with the outlet for pyrolyzed composition of the at least one pyrolysis reactor, and an outlet for cooled pyrolyzed composition being connected with the inlet of the at least one distillation column, wherein the cooler is embodied so as to allow to cool the pyrolyzed composition with a cooling rate of at least 400°C/minute to a temperature of at most 200°C and preferably of 40 to 200°C. Good results are in particular obtained, when the cooler is a spray condenser and/or a shell and tube heat exchanger.

In a further development of the idea of the present invention, it is suggested that the plant comprises two distillation columns, wherein the first distillation column comprises an inlet being connected with the outlet for pyrolyzed composition of the at least one pyrolysis reactor, an overhead outlet and a bottom outlet, and wherein the second distillation column comprises an inlet being connected with the bottom outlet of the first distillation column, an overhead outlet for purified styrene and a bottom outlet.

In accordance with an alternative embodiment of the present invention, the plant comprises three distillation columns, wherein at least the first two of the three distillation columns are extractive distillation columns.

In accordance with an alternative embodiment of the present invention, the plant comprises a dividing-wall distillation column comprising an inlet being connected with the outlet for pyrolyzed composition of the at least one pyrolysis reactor, an outlet for an overhead composition, an outlet for a side composition of purified styrene and an outlet for a bottom composition.

Preferably, the dividing-wall distillation column is a middle dividing-wall distillation column, which comprises a dividing wall extending from a point being located below the top of the distillation column at least essentially vertically downwards to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the dividing wall and a second middle subsection being located on the opposite side of the dividing wall.

Good results are in particular obtained, when the dividing wall extends, seen from the bottom to the top of the dividing-wall distillation column, from a point being located at 10 to 45% of the distance from the bottom to the top of the dividing-wall distillation column to a point being located at 50 to 90% of the distance from the bottom to the top of the dividing-wall distillation column and preferably from a point being located at 25 to 40% of the distance from the bottom to the top of the dividing-wall distillation column to a point being located at 60 to 80% of the distance from the bottom to the top of the dividing-wall distillation column. Moreover, it is preferred that the dividing wall extends, seen from the bottom to the top of the dividing-wall distillation column, over 5 to 90%, more preferably over 20 to 80% and most preferably over 30 to 70% of the distance from the bottom to the top of the dividing-wall distillation column.

In a further development of the idea of the present invention, it is suggested that the dividing wall subdivides the middle section of the dividing-wall distillation column, seen in cross-section of the dividing-wall distillation column, in about two equally sized subsections or halves, respectively. Therefore, it is preferred that one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column. For instance, one subsection covers 43% and the other 57% of the total area of the cross-section or both subsections cover each exactly 50% of the total area of the cross-section. If the total area of the cross-section of the middle section varies over the axial length of the middle section, the above numeric values are related to the average total area of the cross-section of the middle section.

In accordance with a further particular preferred embodiment of the present invention, the plant further comprises at least one melt crystallizer comprising an inlet being connected with the outlet for purified styrene of the last distillation column of the at least one distillation column, and an outlet for purified styrene.

It is further preferred that the at least one melt crystallizer is a crystallization block comprising:
at least one dynamic crystallization section comprising one or more dynamic crystallization stages,
at least one static crystallization section comprising one or more static crystallization stages, and
at least two conduits that fluidly couple at least one of the one or more dynamic crystallization stages with at least one of the one or more static crystallization stages.

Specific embodiments in accordance with the present invention are subsequently described with reference to the appended drawings and by examples.
- Fig. 1: is a schematic view of a plant in accordance with one embodiment of the present invention.
- Fig. 2: is a schematic view of a plant in accordance with another embodiment of the present invention.
- Fig. 3: is a schematic view of a plant in accordance with another embodiment of the present invention.

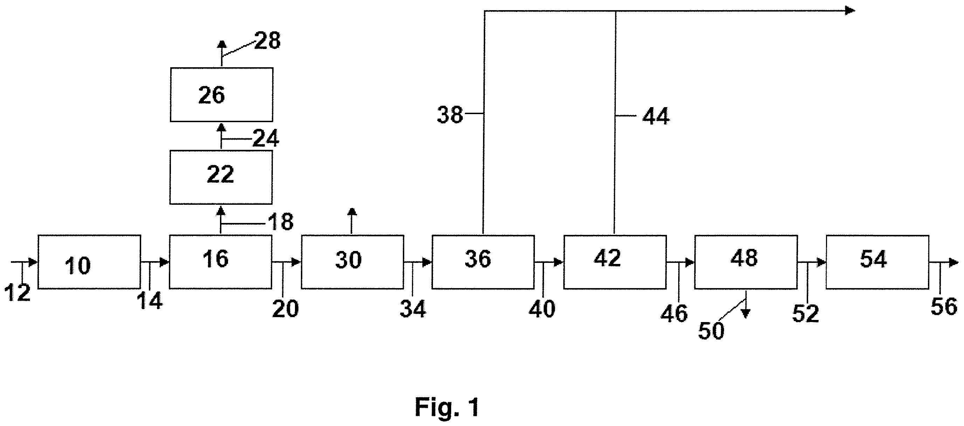

The plant for recycling polystyrene waste containing at least one organic halogen containing compound by converting polystyrene to styrene shown in fig. 1 comprises an extruder 10 with an inlet line 12 for polystyrene waste, such as for expanded polystyrene in the form of chips, flakes, pellets, powder or the like, and on outlet line 14 for a melt of polystyrene waste containing at least one organic halogen containing compound. The outlet line 14 of the extruder 10 is connected with the inlet of a devolatilization vessel 16, which comprises an outlet line 18 for gaseous composition and an outlet line 20 for devolatilized composition. While the outlet line 18 for gaseous composition is connected with an inlet of a washing column 22, which is connected via line 24 with a condenser 26 comprising an outlet line 28 for a liquid light hydrocarbon composition, the outlet line 20 for devolatilized composition of the devolatilization vessel 16 leads into a pyrolysis reactor 30. The pyrolysis reactor 30 comprises an outlet line 32 for residue as well as an outlet line 34 for a pyrolyzed composition, which is connected with a quencher or cooler 36, respectively. The cooler 36 comprises an outlet line 38 for a light hydrocarbon composition as well as an outlet line 40 leading to a first distillation column 42, which comprises an overhead outlet line 44 for a light hydrocarbon composition as well as a bottom outlet line 46, which leads to a second distillation column 48, which comprises a bottom outlet line 50 for C₉₊-hydrocarbons and an overhead outlet line 52 for purified styrene. The outlet line 52 for purified styrene leads into a crystallizer 54, such as a falling film crystallizer, which comprises an outlet line 56 for pure styrene.

During the operation of the plant, polystyrene waste containing at least one organic halogen containing compound, such as for expanded polystyrene in the form of chips, flakes, pellets, powder or the like, is fed into and molten in the extruder 10, before the so obtained polystyrene melt is fed into the devolatilization vessel 16, in which the organic halogen containing compound(s) contained in the polystyrene melt are evaporated and at least partially decomposed to halogen, hydrogen halogenide and/or halogenated hydrocarbons. While the gaseous composition containing the evaporated compounds, such as organic halogen containing compound(s), and decomposition products therefrom, is withdrawn from the devolatilization vessel 16 and is processed in the washing column 22 by contacting it with base, such as a sodium hydroxide solution, and is thereafter condensed in the condenser 26 to a liquid light hydrocarbon composition, the devolatilized composition obtained in the devolatilization vessel 16 is led into the pyrolysis reactor 30 and pyrolyzed therein to a pyrolyzed composition containing styrene, styrene dimer and side-products. The so obtained pyrolyzed composition is then rapidly cooled to a temperature of below 200°C in the cooler 36 and then separated in the two distillation columns 42, 48 into a light hydrocarbon composition containing C₇₋-hydrocarbons being withdrawn via the outlet line 44, into a heavy hydrocarbon composition containing C₉₊-hydrocarbons being withdrawn via the outlet line 50 and into purified styrene being withdrawn via the outlet line 52. The purified styrene is then further purified in the crystallizer 54, from which pure styrene having a styrene content of for instance 99.94% by weight is withdrawn via the outlet line 56.

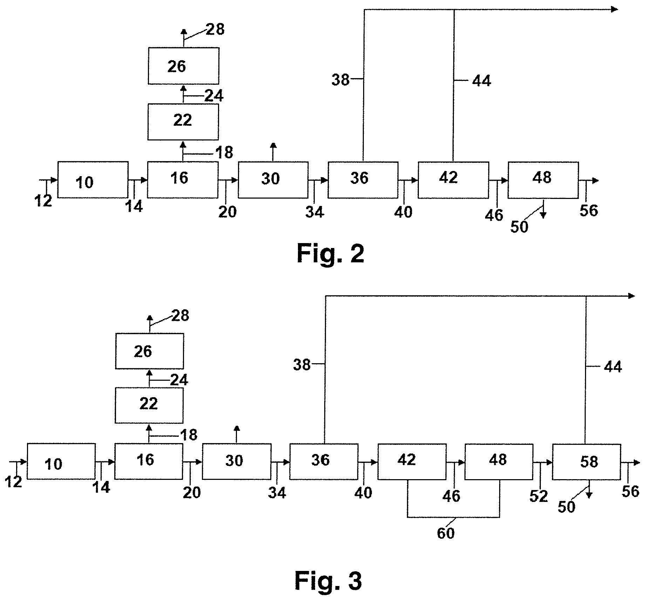

The plant for recycling polystyrene waste containing at least one organic halogen containing compound by converting polystyrene to styrene shown in fig. 2 corresponds to that shown in fig. 1 except that the crystallizer 54 is omitted. The purified styrene obtained with this plant has a styrene content of for instance 99.8% by weight is withdrawn via the outlet line 56.

The plant for recycling polystyrene waste containing at least one organic halogen containing compound by converting polystyrene to styrene shown in fig. 3 corresponds to that shown in fig. 1 except that the crystallizer 54 is replaced by a third distillation column 58 and in that the first two distillations columns 42, 48 are embodied as extractive distillation columns being connected by a solvent recycle line 60. The purified styrene obtained with this plant has a styrene content of for instance 99.8% by weight is withdrawn via the outlet line 56.

### Reference Numeral List

- 10: Extruder
- 12: Inlet line to the extruder
- 14: Outlet line of the extruder
- 16: Devolatilization vessel
- 18: Outlet line for gaseous composition of the devolatilization vessel
- 20: Outlet line for devolatilized composition of the devolatilization vessel
- 22: Washing column
- 24: Line
- 26: Condenser
- 28: Outlet line for a liquid light hydrocarbon composition
- 30: Pyrolysis reactor
- 32: Outlet line for residue of the pyrolysis reactor
- 34: Outlet line for a pyrolyzed composition of the pyrolysis reactor
- 36: Cooler
- 38: Outlet line for a light hydrocarbon composition of the cooler
- 40: Outlet line for a cooled pyrolyzed composition of the cooler
- 42: First distillation column
- 44: Overhead outlet line for a light hydrocarbon composition of the first distillation column
- 46: Bottom outlet line of the first distillation column
- 48: Second distillation column
- 50: Bottom outlet line of the second distillation column
- 52: Overhead outlet line of the second distillation column
- 54: Crystallizer
- 56: Outlet line for pure styrene
- 58: Third distillation column
- 60: Solvent recycle line

## Claims

1. A process for recycling polystyrene waste containing at least one organic halogen containing compound by converting polystyrene to styrene comprising the steps of:
a) providing a melt of polystyrene waste containing at least one organic halogen containing compound,
b) subjecting the melt provided in step a) to at least one devolatilization step so as to obtain a devolatilized composition,
c) subjecting the devolatilized composition obtained in step b) to at least one pyrolysis step so as to obtain a pyrolyzed composition,
d) subjecting the pyrolyzed composition to at least one distillation step so as to obtain a purified styrene composition.

2. The process in accordance with claim 1, wherein the polystyrene waste used in step a) contains 0.5 to 15% by weight of at least one organic halogen containing flame retardant, preferably at least one bromine containing flame retardant, more preferably tetrabromobisphenol A, a brominated polyacrylate, a polybrominated diphenyl ether or a hexabromocyclododecane and most preferably 1,2,5,6,9,10-hexabromocyclododecane.

3. The process in accordance with claim 1 or 2, wherein the at least one devolatilization step b) is performed at a temperature of 180 to 260°C and preferably 210 to 230°C.

4. The process in accordance with any of the preceding claims, wherein the residence time of the melt in the at least one devolatilization step is 5 to 120 minutes and preferably 10 to 30 minutes.

5. The process in accordance with any of the preceding claims, wherein the melt of polystyrene waste containing at least one organic halogen containing compound provided in step a) is mixed in at least one static mixer and preferably in at least one static mixer with heat exchanging capabilities before or during the at least one devolatilization step.

6. The process in accordance with any of the preceding claims, wherein the at least one pyrolysis step is performed at a temperature of 350 to 1,000°C and preferably of 450 to 650°C.

7. The process in accordance with any of the preceding claims, wherein the pyrolyzed composition is cooled with a cooling rate of at least 500°C/minute to a temperature of at most 200°C and preferably of 40 to 200°C, before it is subjected in step d) to at least one distillation step.

8. The process in accordance with any of the preceding claims, wherein i) the pyrolyzed composition is subjected to two distillation steps, wherein the pyrolyzed composition is distilled in a first distillation step into an overhead composition comprising C₇₋-hydrocarbons and into a bottom composition, wherein the bottom composition is distilled in a second distillation step to an overhead composition comprising purified styrene and to a bottom composition containing C₉₊-hydrocarbons, or ii) wherein the pyrolyzed composition is subjected to a distillation step in a dividing-wall distillation column and preferably in a middle dividing-wall distillation column, wherein the pyrolyzed composition is distilled into an overhead composition comprising C₇₋-hydrocarbons, into a side composition of purified styrene and into a bottom composition containing C₉₊-hydrocarbons.

9. The process in accordance with any of the preceding claims, wherein the purified styrene composition obtained in the at least one distillation step is subjected to at least one dynamic melt crystallization step and to at least one static melt crystallization step, wherein preferably first at least one dynamic melt crystallization step is performed and thereafter at least one static melt crystallization step is performed.

10. The process in accordance with claim 9, wherein the purified styrene composition obtained in the at least one distillation step is subjected to one to ten and preferably one to five falling film melt crystallization steps and thereafter to one to ten and preferably one to five static melt crystallization steps.

11. A plant for recycling polystyrene waste containing at least one organic halogen containing compound by converting polystyrene to styrene comprising:
i) at least one devolatilization vessel comprising an inlet for a melt of polystyrene waste containing at least one organic halogen containing compound, an outlet for gaseous composition and an outlet for devolatilized composition,
ii) at least one pyrolysis reactor comprising an inlet being connected with the outlet for devolatilized composition of the at least one devolatilization vessel i) and an outlet for pyrolyzed composition, and
iii) at least one distillation column comprising an inlet being connected directly or indirectly with the outlet for the pyrolyzed composition of the at least one pyrolysis reactor, an overhead outlet and a bottom outlet.

12. The plant in accordance with claim 11, which further comprises an extruder, preferably a single screw extruder or twin-screw extruder, which is arranged upstream of the at least one devolatilization vessel, wherein the extruder comprises an inlet for polystyrene waste containing at least one organic halogen containing compound and an outlet for melt of polystyrene waste containing at least one organic halogen containing compound, wherein the outlet for melt of the extruder is connected with the inlet of the at least one devolatilization vessel.

13. The plant in accordance with claim 11 or 12, which further comprises a cooler comprising an inlet being connected with the outlet for pyrolyzed composition of the at least one pyrolysis reactor, and an outlet for cooled pyrolyzed composition being connected with the inlet of the at least one distillation column, wherein the cooler is preferably a spray condenser.

14. The plant in accordance with any of claims 11 to 13, which i) comprises two distillation columns, wherein the first distillation column comprises an inlet being connected with the outlet for pyrolyzed composition of the at least one pyrolysis reactor, an overhead outlet and a bottom outlet, and wherein the second distillation column comprises an inlet being connected with the bottom outlet of the first distillation column, an overhead outlet for purified styrene and a bottom outlet, or which ii) comprises a dividing-wall distillation column comprising an inlet being connected with the outlet for pyrolyzed composition of the at least one pyrolysis reactor, an outlet for an overhead composition, an outlet for a side composition of purified styrene and an outlet for a bottom composition.

15. The plant in accordance with any of claims 11 to 14, wherein the plant further comprises at least one melt crystallizer comprising an inlet being connected with the outlet for purified styrene of the last distillation column of the at least one distillation column, and an outlet for purified styrene.
